# EUROPEAN PATENT SPECIFICATION

(11) **EP 3 199 169 B1**
(45) Date of publication and mention of the grant of the patent: **08.01.2025**
(21) Application number: 14902879.7
(22) Date of filing: 03.11.2014
(51) Int. Cl.: A61K 9/06, A61K 38/48, C08J 3/075, A61K 35/28, A61K 47/42, C12N 5/0775, A61K 38/01, A61K 38/36, C12N 5/00

(54) **COMPOSITION CONTAINING MESENCHYMAL STEM CELL-HYDROGEL AND METHOD FOR PRODUCING THE COMPOSITION**
ZUSAMMENSETZUNG MIT HYDROGEL AUS MESENCHYMALEN STAMMZELLEN UND VERFAHREN ZUR HERSTELLUNG DER ZUSAMMENSETZUNG
COMPOSITION CONTENANT UN MÉLANGE CELLULES SOUCHES MÉSENCHYMATEUSES - HYDROGEL, ET PROCÉDÉ DE PRODUCTION DE LA COMPOSITION

(30) Priority: 22.09.2014 KR 20140125981
(43) Date of publication of application: 02.08.2017
(73) Proprietor: Anterogen Co., Ltd., Geumcheon-gu, Seoul 153-782 (KR)
(72) Inventor: LEE, Sung-Koo, Seoul 153-782 (KR); KIM, Mihyung, Seoul 153-782 (KR)
(74) Representative: Ström & Gulliksson AB
(86) International application number: PCT/KR2014/010457
(87) International publication number: WO 2016/047849

(56) References cited:
- EP-A1- 3 092 989
- WO-A2-2006/004951
- KR-B1- 100 684 940
- US-A1- 2008 119 385
- US-A1- 2014 227 233
- INOK KIM ET AL: "Fibrin Glue Improves the Therapeutic Effect of MSCs by Sustaining Survival and Paracrine Function", TISSUE ENGINEERING PART A, vol. 19, no. 21-22, 1 November 2013 (2013-11-01), pages 2373 - 2381, XP055155626, ISSN: 1937-3341, DOI: 10.1089/ten.tea.2012.0665
- ISABELLE CATELAS ET AL.: "Human Mesenchymal Stem Cell Proliferation and Osteogenic Differentiation in Fibrin Gels in Vitro", TISSUE ENGINEERING, vol. 12, no. 8, 2006, pages 2385 - 2396, XP055369911
- YUGUO LEI ET AL.: "The spreading, migration and proliferation of mouse mesenchymal stem cells cultured inside hyaluronic acid hydrogels", BIOMATERIALS, vol. 32, 2011, pages 39 - 47, XP027493687, DOI: doi:10.1016/j.biomaterials.2010.08.103

## Description

### [Technical Field]

The present invention relates to a composition including mesenchymal stem cells cultured in hydrogel, more specifically, a adipose tissue-derived mesenchymal stem cell-hydrogel composition and a method of preparing the same. More specifically, the present invention relates to a composition including mesenchymal stem cells cultured in hydrogel, washed, and filled into a syringe, wherein the composition of the present invention may be readily administered without modification and may not need an enzyme treatment in a final process of preparing cells to be transplanted, almost no cell is lost because the composition is washed as hydrogel without a washing process using centrifugation or other methods, and the therapeutic effect is shown immediately after administration to an individual because bioactive substances are patched in pores of the hydrogel.

### [Background Art]

Conventional first-generation stem cell therapeutics are isolated cells obtained by treatment using proteases, such as trypsin or dispase, wherein intercellular bindings and basement membrane proteins of the cells are not maintained because all proteins exposed on cell membranes are nonselectively degraded by the treatment using proteases. In addition, animal-derived substances, such as fetal bovine serum (FBS), added to inactivate a protease is removed by several times of washing-centrifugation process, wherein 5% to 10% of cells are lost by one washing-centrifugation process. Therefore, a cell collection process using a protease is very inefficient.

In addition, the ratio of settled cells is low in a transplantation of isolated single cells to a disease part because transplanted cells are discharged from a target part through diffusion and absorption. Moreover, because mesenchymal stem cells are highly adhesive, the survival rate of isolated single mesenchymal stem cells is very low as it dies within 6 to 24 hours after isolation.

The patent WO 2006/004951 provides a method of de *novo in vivo* synthesis of soft tissues having a predetermined shape and size from adult mesenchymal stem cells in biocompatible scaffolds and a composition prepared by the same method. The patent provides a method of using hydrogel polymer, more specifically, polyethylene glycol diacrylate as a biocompatible scaffold. However, since the composition is for recovery of soft tissue binding, the abovementioned patent only presumes that there should be almost no change in the diameter of the scaffold prepared by using polyethylene glycol diacrylate, and neither provides nor implies a manufacturing method including the filling a cultured substance to a syringe for immediate administration.

The Korean Patent No. 1,289,834 provides a sphincter-regenerative cell therapeutic agent including amniotic fluid-derived stem cells and describes that the cell therapeutic agent may be injected to a hydrogel composite, specifically to an alginate/PF-127/hyaluronic acid to enhance the effect. However, the abovementioned patent does not provide that the process of collecting stem cells may be improved to increase the yield.

The Korean Patent No. 684,940 provides a method of differentiating mesenchymal stem cells to chondrocytes, more specifically, a method of fixing mesenchymal stem cells at a composite scaffold including fibrin/Ha for culturing, wherein fibrin/Ha is a biodegradable polymer. However, the abovementioned patent only provides that the differentiation of mesenchymal stem cells to chondrocytes may be enhanced when fibrin/Ha is used as a scaffold even without adding TGF-beta, w is added in a conventional method of differentiating cells.

In conventional methods, intercellular bindings and basement membrane proteins may be damaged by an enzyme treatment performed in a final process of preparing transplanted cells, cells may be lost in each of cell collection, washing, vial filling, and syringe filling using materials contained in a vial, and most bioactive substances synthesized during cell culture, such as collagen, are eliminated as only single cells are collected and injected after an enzyme treatment.

The present invention provides a composition including mesenchymal stem cells cultured in hydrogel, washed, and filled into a syringe, wherein the composition of the present invention may be readily administered without modification and may not need an enzyme treatment in a final process of preparing cells to be transplanted, almost no cell is lost because the composition is washed as hydrogel without an washing process using centrifugation or other methods, and the therapeutic effect is shown immediately after administration to an individual because bioactive substances are patched in pores of the hydrogel.

### [Citation List]

### [Patent Literature]

WO2006/004951
Korean Patent No. 1,289,834
Korean Patent No. 684,940
US2014/227233 A1

### [Non Patent Literature]

Wu X, Ren J, Li J., Cytotheray, 2012 May; 14(5):555-62
Liao HT. et al., J. Trauma., 2011 Jan; 70(1): 228-37
Wang K. et al., Tissue Eng Part A., 2012 Dec; 18(23-24): 2507-17
Fang H. et al., J Huazhong Univ Sci Technolog Med Sci., 2004;24(3): 272-4
Inok Kim et al., Tissue Eng Part A., 2013; 19(21-22): 2373-81
CATELAS I. et al., tissue engineering 2006; 12 (8); 2385-2396.

### [Summary of Invention]

### [Technical Problem]

The purpose of the present invention is to prepare mesenchymal stem cells separated from adipose tissues and to use them for disease treatment. The present invention provides an improved method of preparing a cell composition to 1) prevent loss of cells in a process of collecting cells cultured in a sufficient amount, 2) prevent damage to cells by protease treatment, and 3) minimize cell loss at a target part; and a composition prepared by the method. The cell composition prepared by the improved method is provided after filling the composition to a syringe to make the utilization easy. The cell-hydrogel prepared and filled by the method provided by the present invention may be applied to all therapeutic agents that may be locally administered by using a 20 to 25-gauge needle. The invention is defined by the appended claims.

### [Solution to Problem]

The present disclosure provides a method of culturing adipose tissue-derived mesenchymal stem cells in a hydrogel and collecting stem cell-hydrogel by using a syringe.

In the composition for disease treatment of the present disclosure, the mesenchymal stem cells are positive for CD29, CD44, CD73, CD90, and etc., or may be autologous or allogenic cells positive to CD34 and CD45.

In one example of the present invention, hydrogel is fibrin glue, wherein the concentration of fibrinogen constituting the fibrin glue is from 0.9 to 1.6 mg/mL.

In one example of the present invention, 100,000 to 2,000,000 stem cells are mixed with 1 mL of hydrogel, 1 to 2 mL of the resulting mixture is added to 12 to 24-well plate to produce a gel, and a medium is added to the gel to culture for three to six days. The medium may be a Dulbecco's Modified Eagle's Medium (DMEM) including 10% FBS and basic fibroblast growth factor (bFGF) or epidermal growth factor (EGF) provided by a conventional patent (Autologous and Allogenic Adipose Tissue-Derived Stromal Stem Cells Composition for Treatment of Fistula; Patent registration number: 1,328,604) or a DMEM including 1% to 10% human serum albumin and 1% to 50% of a composition prepared by a method provided by a convention patent (Method for Preparation of Mesenchymal Stem Cell Culture Comprising High Concentration of Cell Growth Factors and Composition Prepared Therefrom; Patent application number: 10-2011-0043953) .

### [Advantageous Effects of Invention]

The method of preparing a composition including an adipose tissue-derived mesenchymal stem cell-hydrogel of the present patent is very useful, because no enzyme treatment is needed in a final process of preparing transplanted cells as cells are cultured in a hydrogel, washed, and immediately filled to a syringe for use, almost no cell is lost because the composition is washed as hydrogel without a washing process using centrifugation or other methods, and the therapeutic effect is shown immediately after administration to an individual because bioactive substances are patched in pores of the hydrogel.

More specifically, the method of preparing a composition including an adipose tissue-derived mesenchymal stem cell-hydrogel according to the present invention does not include cell isolation (screening) performed by using a protease, and thus cells may be efficiently obtained by preventing cell loss in a conventional cell collection process. In addition, stem cells obtained by the method provided by the present invention may be administered to a disease part in a highly active state, and the administered cells remain at a target part until all the hydrogel is degraded and absorbed completely to show a therapeutic effect continuously. In addition, extracellular matrix secreted from mesenchymal stem cells during culture, such as collagen, laminin, fibronectin, elastin, is completely present as being patched in pores of the hydrogel, and thus the therapeutic capability of the composition of the present invention is significantly higher than that of conventional therapeutic agents, and treatment period may be reduced by using the composition of the present invention.

Experimental results showed that the adipose tissue-derived mesenchymal stem cell-hydrogel of the present invention has a cell survival period significantly longer than that of conventional stem cell therapeutic agents because the stem cells survive more than one week in the hydrogel and maintain the shape of fibroblast, and may enhance healing of wounds because various growth factors and cytokines enhancing cell growth and angiogenesis are continuously secreted for one week, various kinds of extracellular matrix are secreted in a considerable amount, and the secreted extracellular matrix remains in the hydrogel to provide various kinds of matrix when transplanted to a body. In addition, the composition of the present invention does not cause an immune response but inhibits tumor necrosis factor (TNF)-alpha to mitigate inflammation to help healing of wounds, wherein TNF-alpha is an inflammatory substance secreted from activated immune cells.

### [Brief Description of Drawings]

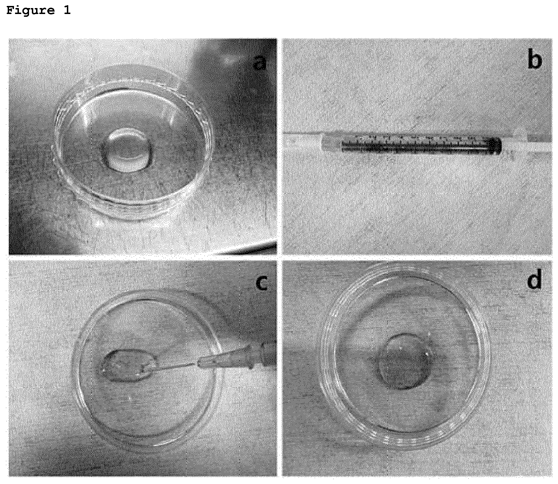

[Figure 1] Figure 1 is an image of a adipose tissue-derived mesenchymal stem cell-hydrogel. Figure 1a shows a cell-hydrogel formed after a cell culture, Figure 1b shows a syringe filled with the cell-hydrogel shown in Figure 1a, and Figures 1c and 1d show the injection of the cell-hydrogel filled into a syringe through a needle and the maintenance of the cell shape following the injection.

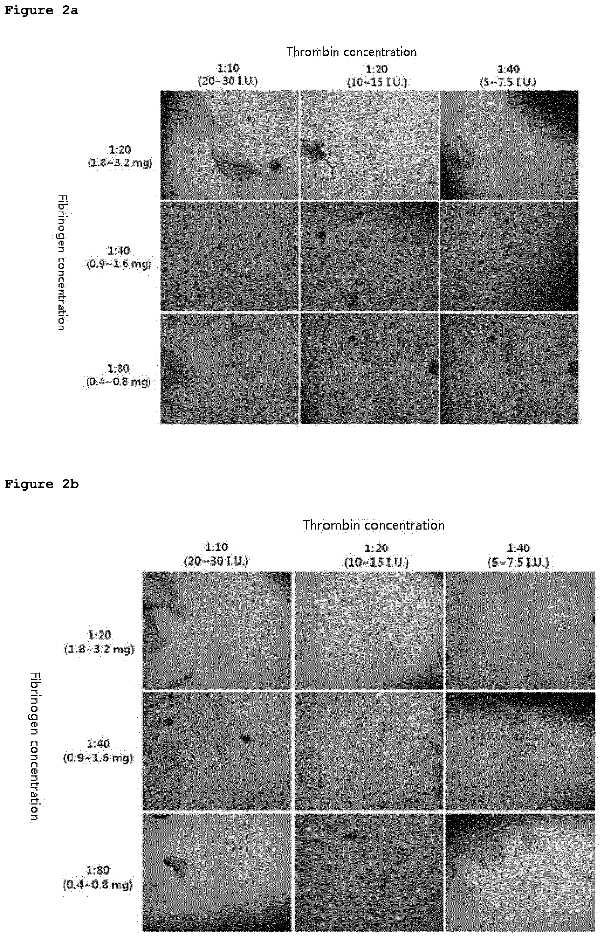

[Figure 2] Figure 2a shows a microscopic image (100 magnifications) of a stem cell-hydrogel filled into a syringe and immediately injected by using a 23-gauge needle, wherein the stem cell-hydrogel was prepared by culturing adipose tissue-derived mesenchymal stem cells mixed with a fibrin gel prepared through a stepwise dilution of fibrinogen and a thrombin solution. The dilution factor and the concentration of fibrinogen and thrombin in the finally prepared hydrogel are shown.
Figure 2b is a microscopic image (100 magnifications) of a stem cell-hydrogel filled into a syringe, kept at 37°C, the human body temperature, for 24 hours, and then injected by using a 23-gauge needle. The dilution factor and the concentration of fibrinogen and thrombin in the finally prepared hydrogel are shown.

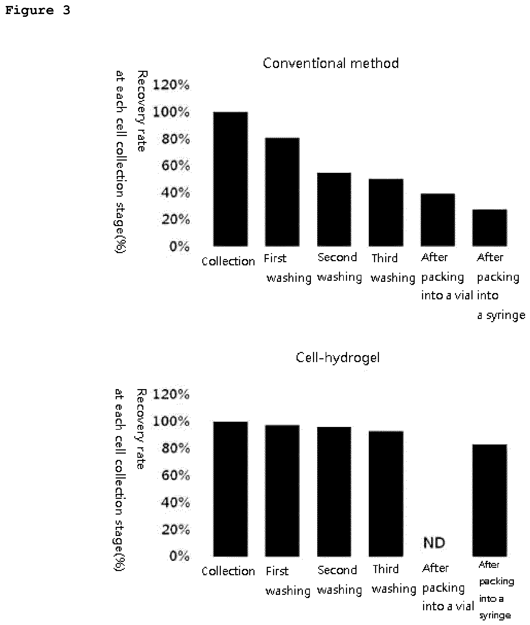

[Figure 3] Figure 3 is a plot showing a cell recovery rate of the cells undergoing a secondary culture by a conventional method at cell collecting, washing, vial filling, and syringe filling and that of the cells undergoing culture in a hydrogel at cell collecting, washing, and syringe filling.

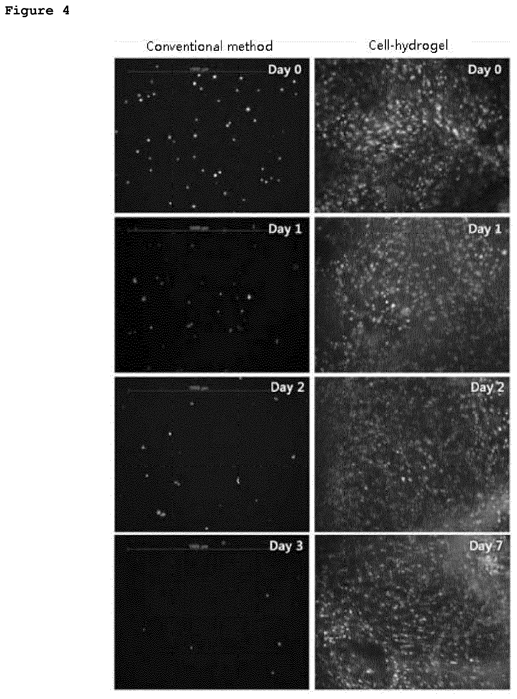

[Figure 4] Figure 4 is an AO/EtBr staining image showing the survival of the cells filled into a vial after a secondary culture by a conventional method (Comparative Example 1) and the cell-hydrogel prepared by culturing cells in a hydrogel and filled into a syringe (Example 3).

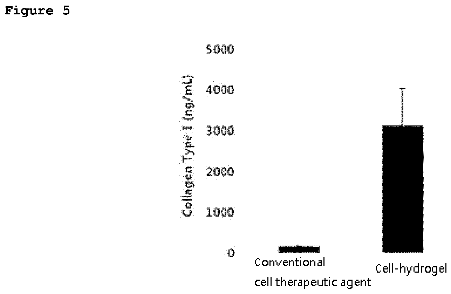

[Figure 5] Figure 5 is a plot showing the quantity of extracellular matrix collagen Type I included in a cell suspension prepared by a conventional method through a secondary culture, collecting, washing and filling (Comparative example 1), and in a cell-hydrogel prepared by culture in a hydrogel, washing, and syringe filling (Example 3), wherein collagen Type I was analyzed by an enzyme-linked immunosorbent assay (ELISA).

### [Description of Embodiments]

The present invention provides a method of preparing a composition including a adipose tissue-derived mesenchymal stem cell-hydrogel as defined in the claims.

The present invention provides a composition including mesenchymal stem cells cultured in hydrogel, washed, and then immediately filled into a syringe, wherein the composition of the present invention does not need an enzyme treatment in a final process of preparing cells to be transplanted, almost no cell is lost because the composition is washed as hydrogel without an washing process using centrifugation or other methods, and the therapeutic effect is shown immediately after administration to an individual because bioactive substances are patched in pores of the hydrogel.

The present invention is described more specifically below.

The present invention relates to a method of preparing a mesenchymal stem cell-hydrogel composition, wherein the method includes (a) culturing of adipose tissue-derived mesenchymal stem cells; (b) forming a gel by mixing the cultured adipose tissue-derived mesenchymal stem cells and a hydrogel solution; and (c) culturing the gel and(d) filling the gel of step (c) into an ampoule, a vial, or a syringe, wherein the hydrogel is fibrin glue, wherein the fibrin glue includes fibrinogen of a concentration from 0.9 to 1.6 mg/mL.

In one aspect, one or a composite of two or more selected from the hydrogel group consisting of fibrin glue, hyaluronic acid, gelatin, collagen, alginic acid, chitosan, cellulose, pectin, a 2-hydroxyethyl methacrylate derivative or a copolymer thereof, polyethylene oxide, and polyvinyl alcohol may be used as a hydrogel, more specifically, one or a composite of two or more selected from the group consisting of fibrin glue, hyaluronic acid, gelatin, collagen, alginic acid, cellulose, and pectin may be used, and more specifically, fibrin glue may be used.

In one embodiment of the present invention, the fibrin glue may include fibrinogen of a concentration from 0.9 to 1.6 mg/mL. An experimental result showed that cells did not grow well or cells were separated from a hydrogel when a final concentration of fibrin glue was out of the range described above.

In one aspect, the fibrin glue may include thrombin of a concentration from 1 to 300 I.U./mL, specifically, from 5 to 30 I.U./mL, more specifically, from 10 to 15 I.U./mL. No problem occurs in cell growth and cell-hydrogel adhesiveness within the range described above.

In one embodiment of the present invention, the method of the present invention includes filling of the mesenchymal stem cell-hydrogel composition to an ampoule, a vial, or a syringe following (c). A adipose tissue-derived mesenchymal stem cell-hydrogel composition prepared by using the preparation method of the present invention may be locally administered by using a 20 to 25-gauge syringe immediately after the preparation.

In one embodiment of the present invention, the number of cells counted in the mesenchymal stem cell-hydrogel filled into an ampoule, a vial, or a syringe in (d) may be 70% or more, more specifically, 80% or more of the number of counted mesenchymal stem cells collected in (c) .

An experimental result showed that about 70% of cells prepared by a conventional method were lost after being filled into a syringe but only 20% or less of cells were lost when the preparation method of the present invention was used, indicating that the cell loss rate of the method provided by the present invention was significantly lower than that of a conventional method.

The present invention provides a cell therapeutic agent including as an active ingredient a mesenchymal stem cell-hydrogel composition prepared by the method.

The term "cell therapeutic agent" used herein refers to a medical product used for the purpose of treatment, diagnosis and prevention, which contains a cell or tissue prepared through isolation from humans, culture and specific operation. Specifically, it refers to a medical product used for the purpose of treatment, diagnosis and prevention through a series of behaviors of in vitro multiplying and sorting living autologous, allogenic and xenogenic cells or changing the biological characteristics of cells by other means for the purpose of restoring the functions of cells and tissues. Cell therapeutic agents are broadly divided into somatic cell therapeutic agents and stem cell therapeutic agents according to the differentiation level of cells. More specifically, the present invention relates to a adipose tissue-derived mesenchymal stem cell therapeutic agent.

The term "subject" used herein refers to all kinds of animals, including humans, already having or likely to have a disease that may be prevented or treated by the administration of the cell therapeutic agent of the present invention.

### [Examples]

Hereinafter, the present invention will be described in further detail with reference to examples.

### Example 1. Method of culturing adipose tissue-derived mesenchymal stem cells

A adipose tissue may usually be obtained by liposuction, but the method of obtaining a adipose tissue is not limited thereto.

Adipose tissue-derived mesenchymal stem cells were separated from adipose tissue obtained by liposuction by the following method. The adipose tissue was washed with the same volume of PBS for three or four times to remove blood. A collagenase solution of a volume same as the adipose tissue was added to the adipose tissue and kept in a water bath at 37°C for reaction. The resulting mixture was transferred to a centrifugation tube and underwent centrifugation at 20°C and 1500 rpm for ten minutes. The supernatant lipid layer was removed, and the bottom collagenase solution layer was carefully separated not to be shaken. A substrate medium was added to the separated collagenase solution layer and suspended, and the resulting mixture underwent centrifugation at 20°C and 1200 rpm for five minutes. The subsiding matter was a stroma-vessel fraction, from which the supernatant was removed. The stroma-vessel fraction was suspended in a substrate medium and then the suspension was inoculated to a culture vessel. Culturing was performed at 37°C in a 5% CO₂ incubator for 24 hours. After removing the culture solution, the cells were washed with PBS, and then proliferated by using a substrate medium, a growth medium prepared by adding 1 ng/ml bFGF to a substrate medium, or a medium prepared by adding 5 ng/ml EGF to a substrate medium. When the adipose tissue-derived mesenchymal stem cells grew to a volume of 80% to 90% of the culture vessel, the cells were treated with trypsin, separated as single cells, and then collected.

### Example 2. Determination of fibrin glue hydrogel concentration

A thrombin solution was added to the adipose tissue-derived mesenchymal stem cells of a concentration of 5 × 10⁵/mL obtained in Example 1 at ratios of 1:10, 1:20, and 1:40. Fibrinogen (71.5 to 126.5 mg/mL) was prepared by diluting at dilution factors of 1:20, 1:40, and 1:80. A Duploject syringe system was used to add 500 to 700 uL of the cell suspensions containing fibrinogen and thrombin to each well of a 12-well plate. After the gel was completely solidified, a culture medium containing 10% FBS and 1 ng/mL bFGF was added, and the mixture was cultured at 37°C in a 5% CO₂ incubator for five days. After washing the cells with DMEM for three times, DMEM containing 1 ng/mL bFGF was added, and the resulting mixture was cultured at 37°C in a 5% CO₂ incubator for about 12 hours. After removing the supernatant, a 1 mL-syringe was used to collect a cell-hydrogel. A 23-gauge needle was connected to the syringe to push the cell-hydrogel to a dish, and the cell-hydrogel was observed with an optical microscope.

Figure 1a is an image of the cell-hydrogel transferred to a dish after culturing at a 12-well plate, indicating that the formed hydrogel was maintained during the culturing period.

Figure 1b is an image of the cell-hydrogel filled into a syringe, indicating that the gel state was maintained even after being filled into the syringe.

Figure 1c shows the injection of the cell-hydrogel filled into a syringe through a 23-gauge needle, and Figure 1d shows that the hydrogel shape of the cell-hydrogel was maintained even after being injected through the 23-gauge needle

Figure 2a shows a microscopic image of the cell-hydrogels prepared at different ratios, and then filled into a syringe and injected to a plate by using a 23-gauge needle. The cell growth was better, as the fibrin glue dilution factor was higher. The cell growth was good and the cells and the fibrin glue hydrogel were well mixed with each other when the final fibrin glue concentration was from 0.9 to 1.6 mg/mL or from 0.4 to 0.8 mg/mL. No significant difference depending on the thrombin concentration was found.

Figure 2b is a microscopic image of a cell-hydrogel prepared at different ratios, filled into a syringe, kept at 37°C, the human body temperature, for 24 hours, and then injected to a plate by using a 23-gauge needle. The cells and the hydrogel were separated from each other almost completely at a final fibrinogen concentration from 1.8 to 3.2 mg/mL and from 0.4 to 0.8 mg/mL, but the cells and the hydrogel were well mixed with each other for up to 24 hours at a final fibrinogen concentration from 0.9 to 1.6 mg/mL. No significant difference depending on the thrombin concentration was found.

### Example 3. Preparation of cell-hydrogel culture solution

A thrombin solution was added to the adipose tissue-derived mesenchymal stem cells of a concentration of 5 × 10⁵/mL obtained in Example 1 at a ratio of 1:20. Fibrinogen was prepared by diluting at a dilution factor of 1:40. Then, the cells were cultured, washed, and filled into a syringe according to Example 2.

### Comparative Example 1. Preparation by using a conventional cell therapeutic agent culture method

The following method was used as a conventional cell therapeutic agent culture method.

Adipose tissue-derived mesenchymal stem cells were cultured in a culture vessel. When the cells grew to a volume of about 80% of the culture vessel, the cells were separated as single cells by adding trypsin-EDTA. After deactivating enzymes by adding DMEM containing FBS, the cells were collected to a centrifugation tube and underwent centrifugation at 1500 rpm. After removing the supernatant, DMEM was added to suspend the cells in order to remove the FMS. Then, the cells again underwent centrifugation at 1500 rpm and the resulting supernatant was removed. The cells were washed for a total of three times, suspended in the final washing with a cell suspension agent of an appropriate volume, and then filled to a vial. The filled cells were transferred to a 1 ml syringe connected with a 23-gauge needle.

### Experimental Example 1. Test of measuring cell recovery rate

A triple expression enzyme was added to the cell-hydrogel at each stage of Example 3 to dissolve fibrin glue, and the number of cells was measured. Similarly, the number of cells was measured at each stage of Comparative Example 1.

Figure 3a is a plot showing the recovery rate of the cells at each stage of the conventional method of preparing the cell therapeutic agent (Comparative Example 1) in comparison with the initial cell collection stage (trypsin-EDTA deactivation), indicating that the cell recovery rate was decreased by 10 to 25% at each stage and only about 300 of the cells were recovered after the cells were finally filled to a syringe in comparison with the initial collection stage.

Figure 3b is a plot showing the recovery rate of the cells at each stage of the preparation method of the present invention (Example 3) in comparison with the initial cell collection stage (after completion of cell culture), indicating that the cell recovery rate was decreased by 1 to 5% at each stage and more than 80% of the cells were recovered after the cells were finally filled to a syringe.

### Experimental Example 2. Survival rate and shape of cells cultured in a cell-hydrogel

The survival rate of the cells cultured in a hydrogel according to Example 3, filled into a 1 mL syringe, and kept at room temperature was measured after 0, 24, and 48 hours and seven days by staining the cells with acridin orange/etidium bromide. The cells prepared by a conventional method of Example 3 and filled to a vial were used as controls.

As shown in Figure 4, the cells prepared by the conventional method and filled to a vial maintained a high survival rate of 95% or higher at Hour 0, but the cells failed to maintain the shape or were aggregated with each other on Hour 24 as the cell membrane was destroyed due to cell death, and almost no cell was observed on Hour 72 as all the cells were dead. On the other hand, the cells cultured in the cell-hydrogel survived until Day 7, and the shape of the fibroblast was maintained.

### Experimental Example 3. Extracellular matrix included in cell-hydrogel

The cell-hydrogel prepared by culturing cells in a hydrogel and filling into a 1 ml syringe according to Example 3 was dissolved by treating with an enzyme, and the quantity of collagen Type I was analyzed by ELISA. A cell suspension prepared by culturing cells by a conventional method of Example 3 and packing into a 1 ml syringe was used as a control.

As shown in Figure 5, almost no collagen was included in the cell suspension prepared by culturing cells by the conventional preparation method of a cell therapeutic agent, but 3 ug/mL or more collagen was included in the cell-hydrogel.

### [Industrial Applicability]

The adipose tissue-derived mesenchymal stem cell-hydrogel composition provided by the present invention has high industrial applicability, because the cells are cultured in hydrogel, washed, and immediately filled into a syringe for use and thus may not need an enzyme treatment in a final process of preparing transplanted cells, almost no cell is lost because the composition is washed as hydrogel without an washing process using centrifugation or other methods, and the therapeutic effect is shown immediately after administration to an individual because bioactive substances are patched in pores of the hydrogel.

## Claims

1. A method of preparing a mesenchymal stem cell-hydrogel composition, wherein the method includes the steps of:
(a) culturing of adipose tissue-derived mesenchymal stem cells;
(b) mixing the cultured adipose tissue-derived mesenchymal stem cells and a hydrogel solution to form a gel;
(c) culturing the gel; and
(d) filling the gel of step (c) into an ampoule, a vial, or a syringe,
wherein the hydrogel is fibrin glue, wherein the fibrin glue includes fibrinogen of a concentration from 0.9 to 1.6 mg/mL.

2. The method of preparing a mesenchymal stem cell-hydrogel composition of claim 1, wherein the fibrin glue includes thrombin of a concentration from 1 to 300 I.U./mL.

3. The method of preparing a mesenchymal stem cell-hydrogel composition of claim 1, wherein the fibrin glue includes thrombin of a concentration from 10 to 15 I.U./mL.

4. The method of preparing a mesenchymal stem cell-hydrogel composition of claim 1, wherein the number of cells counted in the mesenchymal stem cell-hydrogel filled into an ampoule, a vial, or a syringe in Step (d) is 70% or more of the number of cells counted in the mesenchymal stem cells collected in Step (c).

5. The method of preparing a mesenchymal stem cell-hydrogel composition of claim 1, wherein the number of cells counted in the mesenchymal stem cell-hydrogel filled into an ampoule, a vial, or a syringe in Step (d) is 80% or more of the number of cells counted in the mesenchymal stem cells collected in Step (c).

6. A composition for use in cell therapeutic agent comprising mesenchymal stem cell-hydrogel composition prepared by the method of any one of claims 1 to 5.

## Patentansprüche

1. Verfahren zur Herstellung einer Zusammensetzung von mesenchymalen Stammzellen und Hydrogel, wobei das Verfahren die Schritte einschließt:
(a) Kultivieren von aus Fettgewebe gewonnenen mesenchymalen Stammzellen;
(b) Vermischen der kultivierten aus Fettgewebe gewonnenen mesenchymalen Stammzellen und einer Hydrogel-Lösung, um ein Gel zu bilden;
(c) Kultivieren des Gels; und
(d) Abfüllen des Gels von Schritt (c) in eine Ampulle, eine Phiole oder eine Spritze;
wobei das Hydrogel ein Fibrinkleber ist, wobei der Fibrinkleber Fribrinogen einer Konzentration von 0,9 bis 1,6 mg/mL einschließt.

2. Verfahren zur Herstellung einer Zusammensetzung von mesenchymalen Stammzellen und Hydrogel nach Anspruch 1, wobei der Fibrinkleber Thrombin einer Konzentration von 1 bis 300 I.E./mL einschließt.

3. Verfahren zur Herstellung einer Zusammensetzung von mesenchymalen Stammzellen und Hydrogel nach Anspruch 1, wobei der Fibrinkleber Thrombin einer Konzentration von 10 bis 15 I. E./mL einschließt.

4. Verfahren zur Herstellung einer Zusammensetzung von mesenchymalen Stammzellen und Hydrogel nach Anspruch 1, wobei die Anzahl von Zellen, die in den mesenchymalen Stammzellen mit Hydrogel gezählt werden, die in eine Ampulle, eine Phiole oder eine Spritze in Schritt (d) abgefüllt werden, 70 % oder mehr der Anzahl der Zellen beträgt, die in den mesenchymalen Stammzellen gezählt werden, die in Schritt (c) gesammelt werden.

5. Verfahren zur Herstellung einer Zusammensetzung von mesenchymalen Stammzellen und Hydrogel nach Anspruch 1, wobei die Anzahl von Zellen, die in den mesenchymalen Stammzellen mit Hydrogel gezählt werden, die in eine Ampulle, eine Phiole oder eine Spritze in Schritt (d) abgefüllt werden, 80 % oder mehr der Anzahl von Zellen beträgt, die in den mesenchymalen Stammzellen gezählt werden, die in Schritt (c) gesammelt werden.

6. Zusammensetzung zur Verwendung in einem zelltherapeutischen Mittel, umfassend eine Zusammensetzung von mesenchymalen Stammzellen und Hydrogel, hergestellt mit dem Verfahren nach einem der Ansprüche 1 bis 5.

## Revendications

1. Procédé de préparation d'une composition de cellules souches mésenchymateuses et d'hydrogel, dans lequel le procédé comprend les étapes suivantes :
(a) la culture de cellules souches mésenchymateuses dérivées de tissu adipeux ;
(b) le mélange des cellules souches mésenchymateuses dérivées de tissu adipeux cultivées et d'une solution d'hydrogel pour former un gel ;
(c) la culture du gel ; et
(d) l'introduction du gel de l'étape (c) dans une ampoule, un flacon ou une seringue,
dans lequel l'hydrogel est une colle de fibrine, dans lequel la colle de fibrine comprend du fibrinogène à une concentration de 0,9 à 1,6 mg/ml.

2. Procédé de préparation d'une composition de cellules souches mésenchymateuses et d'hydrogel selon la revendication 1, dans lequel la colle de fibrine comprend de la thrombine à une concentration de 1 à 300 Ul/ml.

3. Procédé de préparation d'une composition de cellules souches mésenchymateuses et d'hydrogel selon la revendication 1, dans lequel la colle de fibrine comprend de la thrombine à une concentration de 10 à 15 Ul/ml.

4. Procédé de préparation d'une composition de cellules souches mésenchymateuses et d'hydrogel selon la revendication 1, dans lequel le nombre de cellules comptées dans les cellules souches mésenchymateuses avec hydrogel introduites dans une ampoule, un flacon ou une seringue dans l'étape (d) est de 70 % ou plus du nombre de cellules comptées dans les cellules souches mésenchymateuses collectées dans l'étape (c).

5. Procédé de préparation d'une composition de cellules souches mésenchymateuses et d'hydrogel selon la revendication 1, dans lequel le nombre de cellules comptées dans les cellules souches mésenchymateuses avec hydrogel introduites dans une ampoule, un flacon ou une seringue dans l'étape (d) est de 80 % ou plus du nombre de cellules comptées dans les cellules souches mésenchymateuses collectées dans l'étape (c).

6. Composition pour une utilisation dans un agent thérapeutique cellulaire comprenant une composition de cellules souches mésenchymateuses et d'hydrogel préparée par le procédé selon l'une quelconque des revendications 1 à 5.
